# EUROPEAN PATENT APPLICATION

(11) **EP 4 768 471 A1**
(43) Date of publication of application: **01.07.2026**
(21) Application number: 24867202.4
(22) Date of filing: 19.08.2024
(51) Int. Cl.: C07D 209/42, A61K 31/4439, A61P 19/06

(54) **SIX-MEMBERED AND FIVE-MEMBERED FUSED RING DERIVATIVE, AND PREPARATION METHOD THEREFOR AND USE THEREOF**

(30) Priority: 19.09.2023 CN 202311205835
(71) Applicant: Guangdong Hengqin Novagains Biopharmaceutical Co., Ltd., Zhuhai, Guangdong 519031 (CN); Xiangbei Welman Pharmaceutical Co., Ltd., Changsha, Hunan 410331 (CN); Guangzhou Xin-Chuangyi Biopharmaceutical Co., Ltd., Guangzhou, Guangdong 510535 (CN); Guangzhou Xin Chuang Yi New Drug R&D Co., Ltd, Guangzhou, Guangdong 510535 (CN)
(72) Inventor: SUN, Tianyu, Guangzhou, Guangdong 510620 (CN); CHU, Jiegen, Guangzhou, Guangdong 510620 (CN)
(74) Representative: Kraus & Lederer PartGmbB
(86) International application number: PCT/CN2024/113097
(87) International publication number: WO 2025/060786

(57) **Abstract**

The present application discloses a six-membered and five-membered fused ring derivative, and a preparation method therefor and a use thereof. The compound of the present application is a uric acid transporter inhibitor which can effectively increase excretion of uric acid in the body. In some embodiments of the present application, compared with similar compounds, the compound of the present application has better safety and/or stronger activity.

## Description

### TECHNICAL FIELD

The present disclosure belongs to the field of medicines, and particularly relates to a six-membered and five-membered fused ring derivative, and a preparation method therefor and use thereof.

### BACKGROUND

Abnormal uric acid metabolism is an inducing factor or risk factor for various diseases such as hyperuricemia and gout.

There are two primary approaches to achieve the goal of lowering the uric acid level in vivo. One is to decrease the production of uric acid, for example, the use of xanthine oxidoreductase inhibitors such as allopurinol and febuxostat. The other is to increase the excretion of uric acid, for example, the use of urate transporter inhibitors such as probenecid, benzbromarone and lesinurad. However, these drugs have limiting factors such as suboptimal uric acid-lowering efficacy and insufficient safety profiles, and thus are unable to meet current clinical needs. Verinurad is a next-generation urate transporter inhibitor currently under development. It exhibits an excellent target-binding ability compared to lesinurad, but has encountered repeated setbacks throughout its clinical development.

Therefore, there is a need to further develop better uric acid-lowering drugs.

### SUMMARY

A first aspect of the present disclosure provides a six-membered and five-membered fused ring derivative. To achieve the above objective, the technical solution used in the present disclosure is as follows.

Provided is a compound of Formula I, or a pharmaceutically acceptable salt thereof, 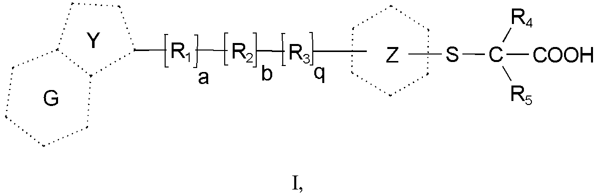 wherein ring G is a 6-membered ring, ring Y is a 5-membered ring, ring Z is a 6-membered ring, and the ring G and the ring Y are fused through two atoms; the ring G, the ring Y or the ring Z is optionally substituted; R₁, R₂ and R₃ are each independently -CH₂- or -S-; a, b and q are each independently 0, 1, 2 or 3, and a, b and q are not all 0; R₄ and R₅ are each independently alkyl or alkoxy, R₄ and R₅ optionally form a ring together or not; and a dashed bond represents a single bond or a double bond, and not both of the two adjacent dashed bonds are double bonds.

In some embodiments of the present disclosure, the ring G, the ring Y or the ring Z is each independently a carbocyclic ring or a heterocyclic ring.

In some embodiments of the present disclosure, the compound of Formula I is represented by Formula I-I: 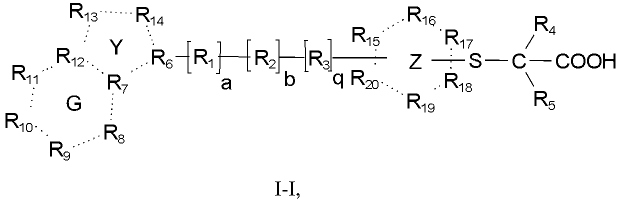 wherein in the Formula I-I, ring G, ring Y, ring Z, R₁, R₂, R₃, R₄, R₅, a, b and q are as defined above; R₆, R₇ and R₁₂ are each independently -C- or -N-; R₈, R₉, R₁₀, R₁₁, R₁₃, R₁₄, R₁₅, R₁₆, R₁₇, R₁₈, R₁₉ and R₂₀ are each independently -C-, -N-, -S- or -O-.

In some embodiments of the present disclosure, at least one of the ring G and the ring Y is a heterocyclic ring.

In some embodiments of the present disclosure, the ring G, the ring Y or the ring Z is each independently a saturated ring or an unsaturated ring.

In some embodiments of the present disclosure, at least one of the ring G and the ring Y is an unsaturated ring.

In some embodiments of the present disclosure, the ring Y is a saturated 5-membered carbocyclic ring. Preferably, the ring Y is cyclopentane.

In some embodiments of the present disclosure, the ring Y is an unsaturated 5-membered carbocyclic ring. Preferably, the ring Y is cyclopentene.

In some embodiments of the present disclosure, the ring Y is a saturated 5-membered heterocyclic ring. Examples of the saturated 5-membered heterocyclic rings include those described in the definitions section of the present disclosure. Preferably, the ring Y is a saturated 5-membered heterocyclic ring containing 1-3 nitrogen atoms. Specific examples include pyrrolidine, pyrazolidine, imidazolidine, etc.

In some embodiments of the present disclosure, the ring Y is an unsaturated 5-membered heterocyclic ring. Examples of the unsaturated 5-membered heterocyclic rings include those described in the definitions section of the present disclosure.

In some embodiments of the present disclosure, the compound of Formula I is represented by Formula I-II: 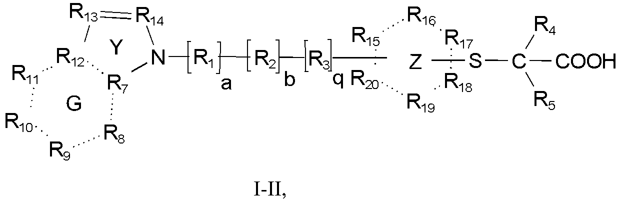 wherein in the Formula I-II, ring G, ring Z, R₁, R₂, R₃, R₄, R₅, R₇, R₈, R₉, R₁₀, R₁₁, R₁₂, R₁₅, R₁₆, R₁₇, R₁₈, R₁₉, R₂₀, a, b and q are as defined above; the ring G, the ring Y or the ring Z is optionally substituted; and R₁₃ and R₁₄ are each independently -C- or -N-.

In some embodiments of the present disclosure, 0 to 2 members selected from the group consisting of R₁₃, R₁₄, R₇ and R₁₂ are -N-.

In some embodiments of the present disclosure, the ring Y is an unsaturated 5-membered heterocyclic ring containing 1-3 nitrogen atoms. Specific examples include pyrrole, imidazole, pyrazole, triazole, etc.

In some embodiments of the present disclosure, the ring Y is selected from the group consisting of Formula Y-1, Formula Y-2, Formula Y-3, Formula Y-4, Formula Y-5, Formula Y-6, Formula Y-7, Formula Y-8, Formula Y-9, Formula Y- 10 and Formula Y-11, wherein a dashed bond represents a single bond or a double bond, and not both of the two adjacent dashed bonds are double bonds,

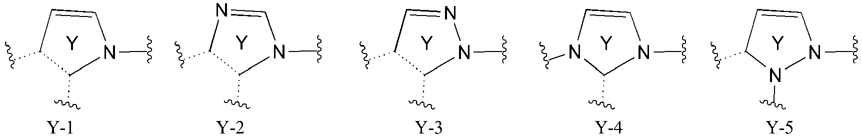

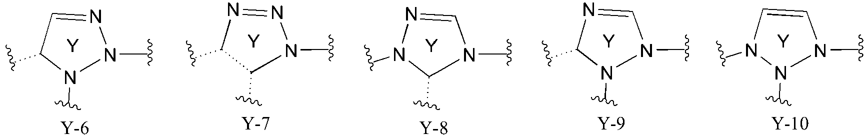

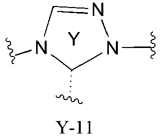

In some embodiments of the present disclosure, the ring G is a saturated 6-membered carbocyclic ring. Preferably, the ring G is cyclohexane.

In some embodiments of the present disclosure, the ring G is a saturated 6-membered heterocyclic ring. Examples of the saturated 6-membered heterocyclic rings include those described in the definitions section of the present disclosure. Preferably, the ring G is piperidine, piperazine, morpholine, etc.

In some embodiments of the present disclosure, the ring G is an unsaturated 6-membered carbocyclic ring. Examples of the unsaturated 6-membered carbocyclic rings include those described in the definitions section of the present disclosure. Preferably, the ring G is a benzene ring.

In some embodiments of the present disclosure, the ring G is an unsaturated 6-membered heterocyclic ring. Examples of the unsaturated 6-membered heterocyclic rings include those described in the definitions section of the present disclosure. Preferably, the ring G is an unsaturated 6-membered heterocyclic ring containing 1-3 nitrogen atoms. Specific examples include pyridine, pyridazine, pyrimidine, pyrazine, triazine, etc.

In some embodiments of the present disclosure, the compound of Formula I is represented by Formula **I-III:** 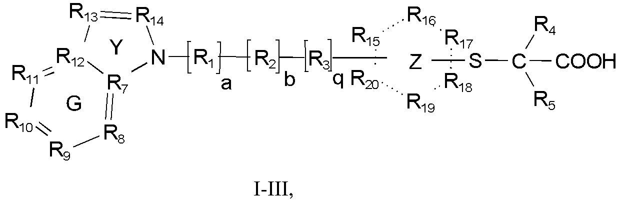 wherein in the Formula I-III, ring Z, R₁, R₂, R₃, R₄, R₅, R₁₅, R₁₆, R₁₇, R₁₈, R₁₉, R₂₀, a, b and q are as defined above, ring G, ring Y or the ring Z may be optionally substituted; and R₇, R₁₂, R₁₃ and R₁₄ are each independently -C- or -N-; and R₈, R₉, R₁₀ and R₁₁ are each independently -C- or -N-.

In some embodiments of the present disclosure, 0 to 3 members selected from the group consisting of R₈, R₉, R₁₀ and R₁₁ are -N-.

In some embodiments of the present disclosure, the ring G is selected from the group consisting of Formula G-1, Formula G-2, Formula G-3, Formula G-4, Formula G-5, Formula G-6, Formula G-7, Formula G-8, Formula G-9, Formula G-10 and Formula G-11,

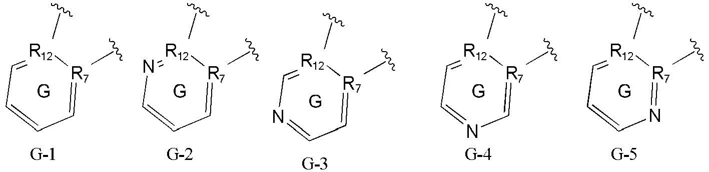

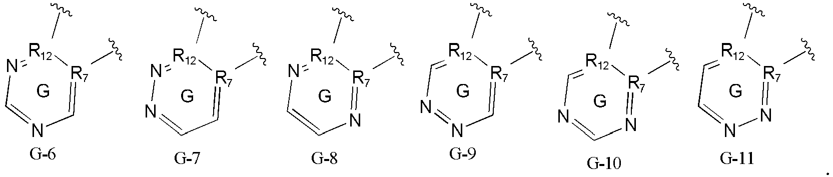

In some embodiments of the present disclosure, the ring Z is an unsaturated 6-membered carbocyclic ring. Examples of the unsaturated 6-membered carbocyclic rings include those described in the definitions section of the present disclosure. Preferably, the ring Z is a benzene ring.

In some embodiments of the present disclosure, the ring Z is an unsaturated 6-membered heterocyclic ring. Examples of the unsaturated 6-membered heterocyclic rings include those described in the definitions section of the present disclosure. Preferably, the ring Z is an unsaturated 6-membered heterocyclic ring containing 1-3 nitrogen atoms. Specific examples include pyridine, pyridazine, pyrimidine, pyrazine, triazine, etc.

In some embodiments of the present disclosure, the compound of Formula I is represented by Formula I-IV: 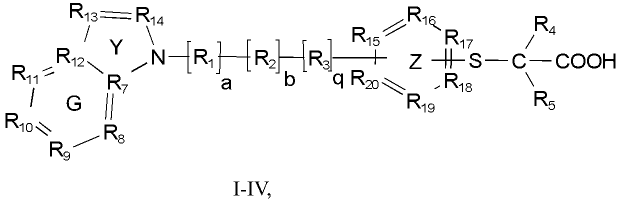 wherein in the Formula I-IV, R₁, R₂, R₃, R₄, R₅, a, b and q are as defined above, ring G, ring Y or ring Z may be optionally substituted; and R₇, R₁₂, R₁₃ and R₁₄ are each independently -C- or -N-; R₈, R₉, R₁₀ and R₁₁ are each independently -C- or -N-; and R₁₅, R₁₆, R₁₇, R₁₈, R₁₉ and R₂₀ are each independently -C- or -N-.

In some embodiments of the present disclosure, 0 to 3 members selected from the group consisting of R₁₅, R₁₆, R₁₇, R₁₈, R₁₉ and R₂₀ are -N-.

In some embodiments of the present disclosure, a is 1, 2 or 3.

In some embodiments of the present disclosure, a is 0.

In some embodiments of the present disclosure, b is 1, 2 or 3.

In some embodiments of the present disclosure, b is 0.

In some embodiments of the present disclosure, q is 1, 2 or 3.

In some embodiments of the present disclosure, q is 0.

In some embodiments of the present disclosure, b is 1, 2 or 3, a is 0, and q is 0.

In some embodiments of the present disclosure, R₁ is -CH₂-.

In some embodiments of the present disclosure, R₁ is -S-.

In some embodiments of the present disclosure, R₂ is -CH₂-.

In some embodiments of the present disclosure, R₂ is -S-.

In some embodiments of the present disclosure, R₃ is -CH₂-.

In some embodiments of the present disclosure, R₃ is -S-.

In some embodiments of the present disclosure, R₁ is -CH₂-, R₂ is -S-, R₃ is -CH₂-, a and q are independently 0, 1, 2 or 3, and b is 1.

In some embodiments of the present disclosure, R₂ is -CH₂-, b is 1, 2 or 3, a is 0, and q is 0.

In some embodiments of the present disclosure, the compound of Formula I is represented by Formula I-V: 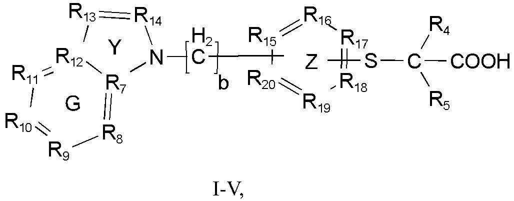 wherein in the Formula I-V, R₄ and R₅ are as defined above, and ring G, ring Y or ring Z may be optionally substituted; R₇, R₁₂, R₁₃ and R₁₄ are each independently -C- or -N-; R₈, R₉, R₁₀ and R₁₁ are each independently -C- or -N-; R₁₅, R₁₆, R₁₇, R₁₈, R₁₉ and R₂₀ are each independently -C- or -N-; and b is 1, 2 or 3.

In some embodiments of the present disclosure, the ring G is substituted. Substituents can be alkyl, alkoxy, halogen, cyano, hydroxyl, or carboxyl, etc. The number of the substituents can be one or more.

In some embodiments of the present disclosure, the ring Y is substituted. Substituents can be alkyl, alkoxy, halogen, cyano, hydroxyl, or carboxyl, etc. The number of the substituents can be one or more.

In some embodiments of the present disclosure, the ring Z is substituted. Substituents can be alkyl, alkoxy, halogen, cyano, hydroxyl, or carboxyl, etc. The number of the substituents can be one or more.

In some embodiments of the present disclosure, the compound of Formula I is represented by Formula I-VI: 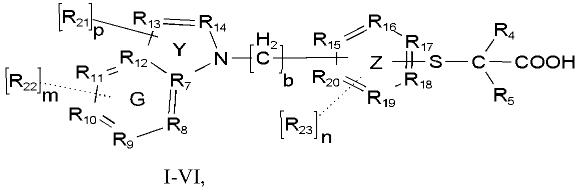 wherein in the Formula I-VI, R₄ and R₅ are as defined above; ring Y is substituted with R₂₁, ring G may be optionally substituted with R₂₂, and ring Z may be optionally substituted with R₂₃; R₇, R₁₂, R₁₃ and R₁₄ are each independently -C- or -N-; R₈, R₉, R₁₀ and R₁₁ are each independently -C- or -N-; R₁₅, R₁₆, R₁₇, R₁₈, R₁₉ and R₂₀ are each independently -C- or -N-; b is 1, 2 or 3; R₂₁ is alkyl, alkoxy, halogen or cyano; R₂₂ and R₂₃ are each independently alkyl, alkoxy or halogen; and p is 1 or 2, and m and n are each independently 0, 1, 2, 3 or 4.

In some embodiments of the present disclosure, the compound of Formula I is represented by Formula I-VII: 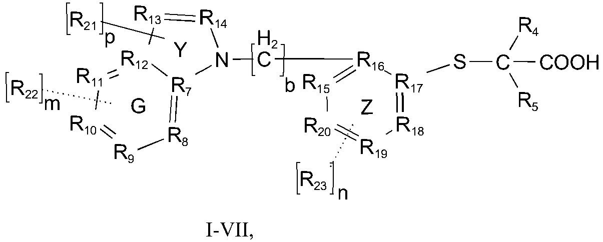 wherein in the formulas I-VII, R₄, R₅, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃, b, p, m and n are as described above.

In some embodiments of the present disclosure, R₄ and R₅ are each independently C₁-C₅ alkyl. Examples of the C₁-C₃ alkyl include those described in the definitions section of the present disclosure. In some embodiments of the present disclosure, R₄ and R₅ form a ring together, the formed ring can be a 3-membered to 6-membered ring such as cyclopropyl, cyclobutyl, cyclopentyl, or cyclohexyl, etc. In some embodiments of the present disclosure, R₄ and R₃ do not form a ring together.

Since the compound of the present disclosure is a carboxylic acid, those skilled in the art can easily prepare the compound into a pharmaceutically acceptable salt. Therefore, the present disclosure includes a pharmaceutically acceptable salt of the compound of the present disclosure.

Since the compound of the present disclosure is a carboxylic acid, those skilled in the art can easily prepare the compound into an ester. Therefore, the present disclosure further includes an ester of the compound of the present disclosure, such as a compound represented by Formula II: 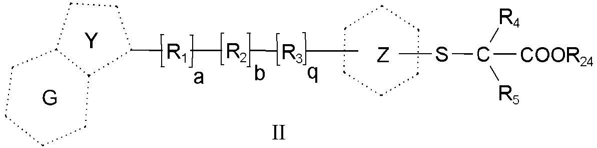 wherein in the Formula II, ring Y, ring G, ring Z, R₁, R₂, R₃, R₄, R₅, a, b and q are as defined above; and R₂₄ is alkyl, preferably C₁-C₃ alkyl.

The carboxylic acids represented by the Formula I-I, Formula I-II, Formula I-III, Formula I-IV, Formula I-V, Formula I-VI and Formula I-VII can also include the above-mentioned R₂₄ group at corresponding positions, for example, 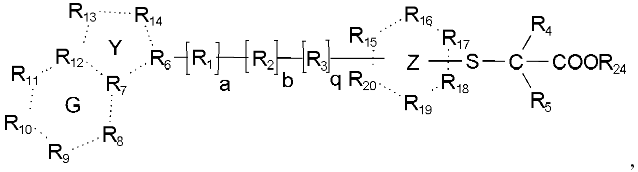 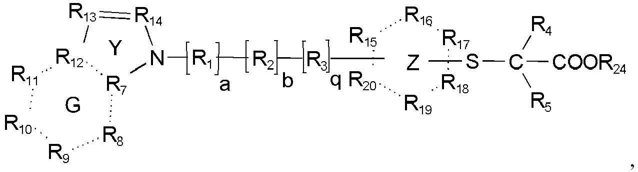 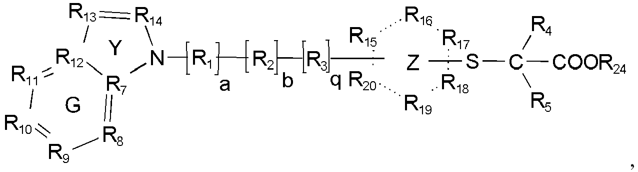 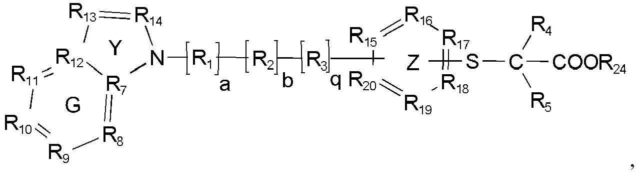 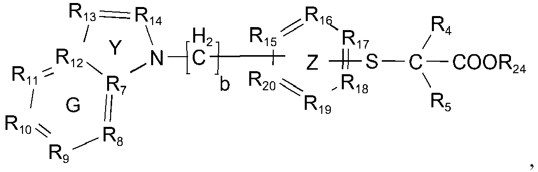 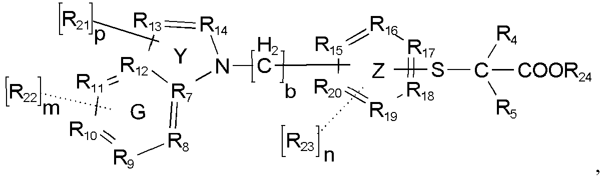 and

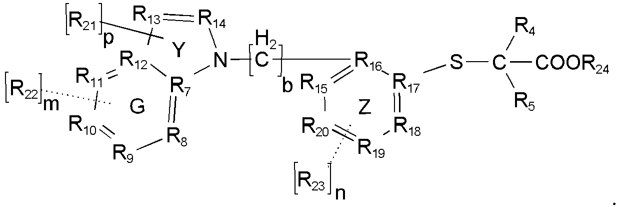

The definition of each ring or each substituent in the present disclosure is as defined above, however, the rings or substituents in each technical solution can be combined at random.

A second aspect of the present disclosure provides a method for preparing the compound of Formula I as described above. To achieve the above objective, the technical solution used in the present disclosure is as follows.

Provided is a method for preparing the compound of Formula I, comprising subjecting a compound of Formula II to hydrogenation reaction as shown in a reaction equation below to obtain the compound of Formula I; 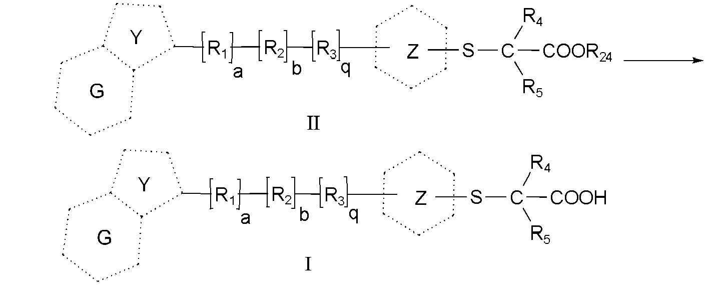 wherein ring Y, ring G, ring Z, R₁, R₂, R₃, R₄, R₅ and R₂₄ are as defined above.

In some embodiments of the present disclosure, provided is a method for preparing the compound of Formula II, comprising reacting a compound of Formula III with a compound of Formula IV as shown in a reaction equation below to obtain the compound of Formula II; 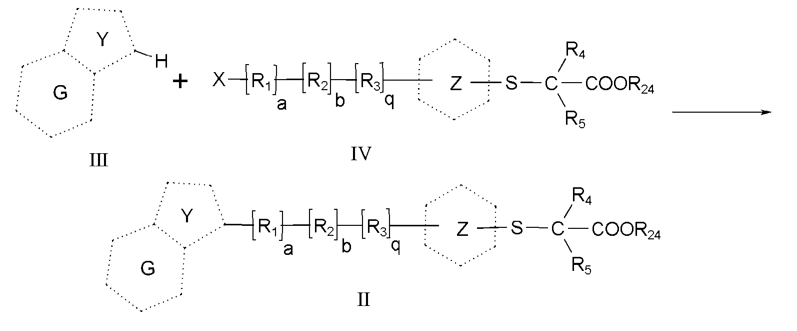 wherein ring Y, ring G, ring Z, R₁, R₂, R₃, R₄, R₅ and R₂₄ are as defined above. In the Formula IV, X represents halogen.

In some embodiments of the present disclosure, provided is a method for preparing the compound of Formula IV, comprising reacting a compound of Formula V as shown in a reaction equation below to obtain the compound of Formula IV; 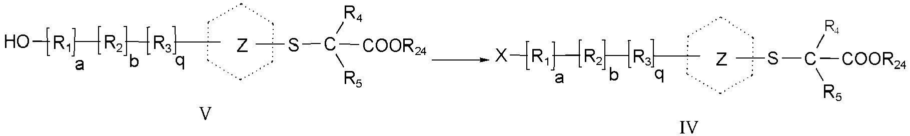 wherein ring Z, R₁, R₂, R₃, R₄, R₅ and R₂₄ are as defined above. In the Formula IV, X represents halogen.

In some embodiments of the present disclosure, provided is a method for preparing the compound of Formula V, comprising reacting a compound of Formula VI as shown in a reaction equation below to obtain the compound of Formula V; 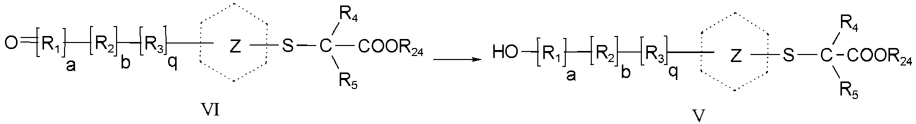 wherein ring Z, R₁, R₂, R₃, R₄, R₅ and R₂₄ are as defined above.

In some embodiments of the present disclosure, provided is a method for preparing the compound of Formula VI, comprising reacting a compound of Formula VII with a compound of Formula VIII as shown in a reaction equation below to obtain the compound of Formula VI; 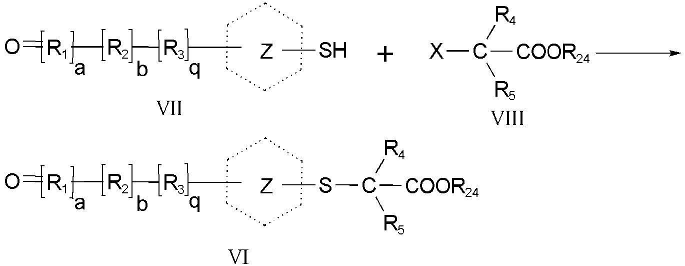 wherein ring Z, R₁, R₂, R₃, R₄, R₅ and R₂₄ are as defined above. In the Formula VIII, X represents halogen.

In some embodiments of the present disclosure, provided is a method for preparing the compound of Formula VII, comprising reacting a compound of Formula IX as shown in a reaction equation below to obtain the compound of Formula VII; 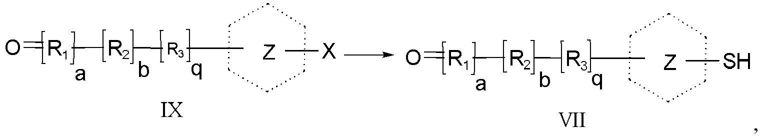 wherein ring Z is as defined above. In the Formula IX, X represents halogen.

Based on the method for preparing the Formula I, the compounds of Formula I-I, Formula I-II, Formula I-III, Formula I-IV, Formula I-V, Formula I-VI and Formula I-VII can be prepared.

A third aspect of the present disclosure provides use of the compound of Formula I. To achieve the above objective, the technical solution used in the present disclosure is as follows.

Provided is use of the compound of Formula I as described above in the preparation of a drug for lowering a uric acid level in vivo; and
use of the compound of Formula I as described above in the preparation of a drug for treating a disease or a symptom associated with an abnormal uric acid level in vivo.

In some embodiments of the present disclosure, the disease is hyperuricemia, gout, or renal failure associated with an abnormal uric acid level, etc.

The compound of the present disclosure is a urate transporter inhibitor which can effectively increase the excretion of uric acid in vivo. In some embodiments of the present disclosure, the compounds of the present disclosure exhibit an improved safety profile compared to similar compounds. In some embodiments of the present disclosure, the compounds of the present disclosure have stronger activity against urate transporter URAT1 compared to similar compounds. These results demonstrate that the six-membered and five-membered fused ring derivative provided in the present disclosure has unexpectedly superior properties compared to the compounds in the prior art.

### DETAILED DESCRIPTION

The present disclosure will be described in detail below with reference to embodiments. It should be understood that the contents of the embodiments are illustrative only and not limiting, i.e., they do not impose any limitation on the contents of the present disclosure.

### Definitions:

The term "5-membered ring" refers to a ring where the total number of atoms constituting the ring is 5.

The term "6-membered ring" refers to a ring where the total number of atoms constituting the ring is 6.

The term "saturated ring" refers to a ring where all bonds formed among the atoms constituting the ring are single bonds.

The term "unsaturated ring" refers to a ring where the bonds formed among the atoms constituting the ring are not all single bonds, but also double bonds.

The term "carbocyclic ring" refers to a ring where all atoms constituting the ring are carbon atoms.

The term "heterocyclic ring" refers to a ring where the atoms constituting the ring are not all carbon atoms, but also other atoms (referred to as heteroatoms). The heteroatom can be one or more selected from the group consisting of sulfur, nitrogen and oxygen. The number of the heteroatoms can be one or more.

The "saturated 6-membered carbocyclic ring" includes cyclohexane.

The "unsaturated 6-membered carbocyclic ring" includes benzene and cyclohexene.

The "saturated 6-membered heterocyclic ring" includes piperidine, tetrahydropyran (oxacyclohexane), tetrahydrothiopyran(thiane), hexahydropyrimidine, hexahydropyrazine (piperazine), hexahydropyridazine, thiane, dithiane, dioxane, morpholine, thiomorpholine, etc.

The "unsaturated 6-membered heterocyclic ring" includes dihydropyridine, tetrahydropyridine, pyridine, dihydropyran, pyran, dihydrothiopyran, thiopyran, dihydropyrimidine, pyrimidine, dihydropyrazine, pyrazine, dihydropyridazine, pyridazine, oxazine, thiazine, triazine, etc.

The "saturated 5-membered carbocyclic ring" includes cyclopentane.

The "unsaturated 5-membered carbocyclic ring" includes cyclopentene.

The "saturated 5-membered heterocyclic ring" includes pyrrolidine, tetrahydrofuran, tetrahydrothiophene, pyrazolidine, imidazolidine, oxazolidine, isoxazolidine, thiazolidine, isothiazolidine, dioxolane, oxathiolane, etc.

The "unsaturated 5-membered heterocyclic ring" includes pyrroline, pyrrole, dihydrofuran, furan, dihydrothiophene, thiophene, dihydropyrazole (pyrazoline), pyrazole, dihydroimidazole (imidazoline), imidazole, dihydrooxazole, oxazole, dihydroisoxazole, isoxazole, dihydrothiazole, thiazole, dihydroisothiazole, isothiazole, triazole, tetrazole, etc.

The term "alkyl" refers to a hydrocarbon group composed entirely of carbon and hydrogen atoms, including linear alkyl, branched alkyl or cycloalkyl; saturated alkyl or unsaturated alkyl; substituted or unsubstituted alkyl, for example, those substituted with halogen, alkyl, alkoxy, hydroxyl, carboxyl, amino and the like. The C₁-C₅ alkyl also includes linear, branched or cyclic C₁-C₅ alkyl, as well as saturated or unsaturated C₁-C₅ alkyl. Specifically, it at least includes: methyl, methylene, ethyl, vinyl, alkynyl, n-propyl, isopropyl, cyclopropyl, propenyl, methyl-substituted vinyl, propynyl, n-butyl, isobutyl, tert-butyl, cyclobutyl, methyl-substituted propyl, butenyl, methyl-substituted propenyl, butynyl, methyl-substituted propynyl, n-pentyl, isopentyl, cyclopentyl, methyl-substituted butyl, dimethyl-substituted propyl, ethyl-substituted propyl, pentenyl, methyl-substituted butenyl, ethyl-substituted propenyl, dimethyl-substituted propenyl, pentynyl, methyl-substituted butynyl, ethyl-substituted propynyl, dimethyl-substituted propynyl, etc., and also includes these groups further substituted with alkyl, halogen or the like.

The "alkoxy" includes linear alkoxy, branched alkoxy or cycloalkyloxy. The "alkoxy" includes saturated alkoxy or unsaturated alkoxy. The "alkoxy" includes substituted or unsubstituted alkoxy, for example, those substituted with halogen, alkyl, alkoxy, hydroxyl, carboxyl, amino or the like.

The "halogen" includes fluorine, chlorine, bromine and iodine.

The "hydroxyl" includes substituted or unsubstituted hydroxyl, for example those substituted with alkyl, alkoxy, carboxyl, amino or the like.

The "carboxyl" includes substituted or unsubstituted carboxyl, for example those substituted with alkyl, amino or the like.

In addition, "-C-" and "-N-" are generic notations. The "-C-" means that a carbon atom at least forms two main bonds, and the formed bonds may be single bonds or double bonds. In addition, this notation omits hydrogen atoms, and thus encompasses cases where the carbon atom is bonded to 0, 1 or 2 hydrogen atoms. The "-N-" means that nitrogen atoms at least form two main bonds, and the formed bonds may be single bonds or double bonds. In addition, this notation omits hydrogen atoms, and thus encompasses cases where the nitrogen atom is bonded to 0 or 1 hydrogen atoms.

### Example 1: Preparation of Compound F-02

### 1.1 Preparation of F201

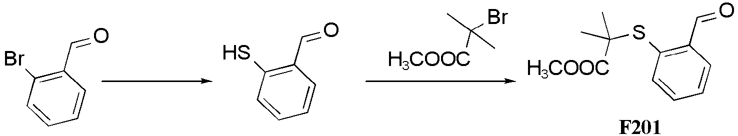

Sodium hydrosulfide hydrate (17.3 g) and dimethylformamide (200 mL) were added to a reaction flask and stirred until dissolved. 2-bromobenzaldehyde (20.8 g) was added, and the reaction mixture was allowed to reaction at 70°C until thin-layer chromatography (TLC) indicated that the starting materials were completely consumed. Then, water, ethyl acetate and acetic acid were added for extraction. An organic phase was separated, washed, dried and concentrated to afford 2-mercaptobenzaldehyde.

Dimethylformamide (100 mL) and potassium carbonate (29.8 g) were added to 2-mercaptobenzaldehyde, and then methyl 2-bromoisobutyrate (23.5 g) was added under stirring to allow for reaction for 2 h at room temperature. Then water and ethyl acetate were added for extraction. An organic phase was separated, washed, dried, concentrated and purified by column chromatography to afford product F201 (15.2 g).

### 1.2 Preparation of F202

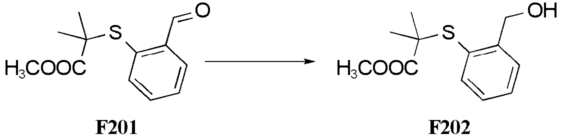

F201 (15.2 g, prepared according to the method in 1.1 as described above) and methanol (100 mL) were added to a reaction flask. Sodium borohydride (2.4 g) was added portionwise at -10°C. After reaction for 0.5 h, the reaction was quenched with water. The resulting reaction product was concentrated under reduced pressure to remove methanol, and then added with water and ethyl acetate for extraction. An organic phase was separated, washed, dried, concentrated and purified by column chromatography to afford product F202 (9 g).

### 1.3 Preparation of F203

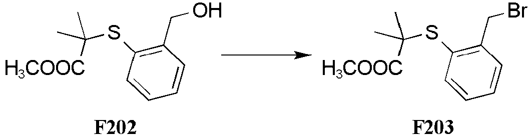

F202 (1.2 g, prepared according to the method in 1.2 as described above) and dichloromethane (13 mL) were added to a reaction flask. Phosphorus tribromide (1.35 g) was added at -10°C. The reaction mixture was allowed to reaction at room temperature until the starting materials was completely consumed. The ethyl acetate and saline were added for extraction. An organic phase was separated, dried and purified by column chromatography to afford product F203.

### 1.4 Preparation of F204

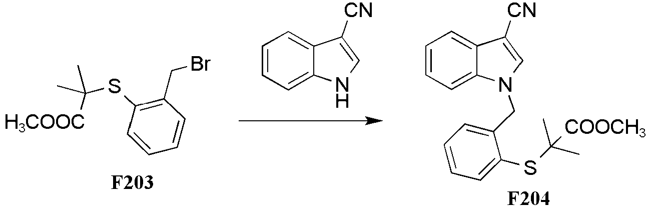

3-cyanoindole (0.736 g), sodium hydroxide (0.4 g) and dimethylformamide (10 mL) were added to a reaction flask and stirred to allow reaction for 1 h at room temperature. F203 (prepared according to the method in 1.3) was then added, and the reaction was continued at room temperature until the starting materials was completely consumed. The resulting reaction product was extracted with ethyl acetate. An organic phase was washed with saline, dried, concentrated and purified by column chromatography to afford product F204 (0.845 g).

### 1.5 Preparation of F-02

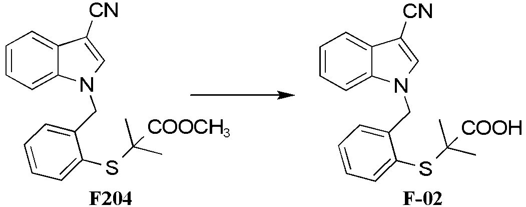

F204 (0.845 g, prepared according to the method in 1.4 as described above), methanol (10 mL), water (2 mL) and lithium hydroxide (0.195 g) were added to a reaction flask and allowed to reaction for 1.5 h at 50°C. Dilute hydrochloric acid was added to adjust the pH to 5. The resulting reaction product was concentrated under reduced pressure. Water and ethyl acetate were added for extraction. An organic phase was separated, dried, concentrated and purified by column chromatography to afford product F-02 (0.660 g). Mass spectrum: [M+H]⁺:351.11; nuclear magnetism: ¹H NMR (400 MHz, DMSO-*d*₆) δ 12.86 (br, 1H), δ 8.33 (s, 1H), 7.71-7.68 (m, 1H), 7.60-7.57 (m, 1H), 7.54-7.51 (m, 1H), 7.33-7.28 (m, 4H), 6.77-6.73 (m, 1H), 5.71 (s,2 H), 1.46 (s, 6H).

### Example 2: Preparation of Compound F-07

### 2.1 Preparation of F704

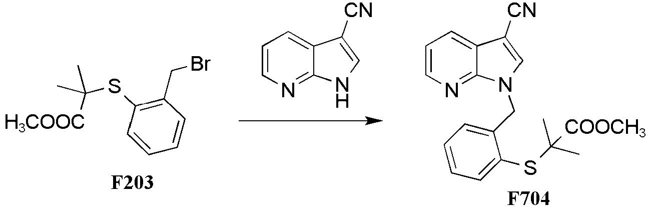

3-cyano-7-azaindole (0.5 g), sodium hydroxide (0.69 g), dimethylformamide (10 mL) and the intermediate F203 (1.58 g, prepared according to the method in Example 1) were added to a reaction flask and allowed to reaction for 0.5 h at room temperature. Then water and ethyl acetate were added for extraction. An organic phase was separated, washed with saline, dried, concentrated and purified by column chromatography to afford product F704 (0.51 g).

### 2.2 Preparation of F-07

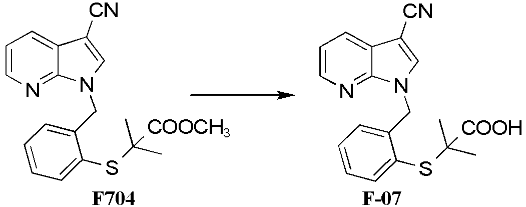

The intermediate F704 (0.9 g, prepared according to the method in 2.1 as described above), tetrahydrofuran (10 mL), methanol (15 mL), water (10 mL) and lithium hydroxide (0.21 g) were added to a reaction flask and allowed to reaction for 4 h at 60°C. Acetic acid was added to adjust the pH to 7. The resulting reaction product was concentrated and extracted with ethyl acetate. An organic phase was separated, concentrated and purified by column chromatography to afford product F-07 (0.640 g). Mass spectrum: [M+H]⁺: 352.13; nuclear magnetism: ¹H NMR (400 MHz, DMSO-d6) δ 12.81 (s, 1H), 8.47 (s, 1H), 8.45 (dd, J = 4.8, 1.5 Hz, 1H), 8.20 (dd, J = 8.0, 1.5 Hz, 1H), 7.58-7.53 (m, 1H), 7.37 (dd, J = 8.0, 4.7 Hz, 1H), 7.34-7.29 (m, 2H), 6.85-6.80 (m, 1H), 5.79 (s, 2H), 1.45 (s, 6H).

### Example 3: Preparation of Compound F-09

### 3.1 Preparation of F901

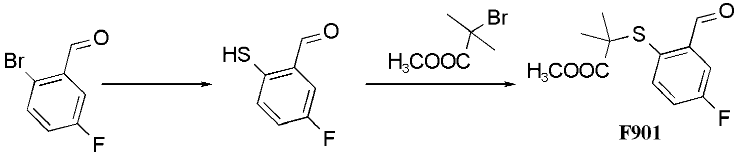

Sodium hydrosulfide (4.4 g), dimethylformamide (100 mL) and potassium carbonate (10.4 g) were added to a reaction flask, and then 5-fluoro-2-bromobenzaldehyde (10.2 g) was added under stirring to allow reaction at 70°C until the starting materials were almost completely consumed. Then, the reaction product was cooled.

Methyl 2-bromoisobutyrate (10 g) was added for reaction for 20 min at room temperature. Then water and ethyl acetate were added for extraction. An organic phase was separated, washed with an aqueous copper acetate solution, washed with saline, dried, concentrated and purified by column chromatography to afford product F901 (4 g).

### 3.2 Preparation of F902

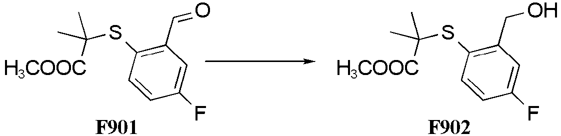

F901 (4 g, prepared according to the method in 3.1 as described above) and methanol (25 mL) were added to a reaction flask, and then sodium borohydride (0.42 g) was added at -10°C. After reaction for 0.5 h, the reaction was quenched with water. The resulting reaction product was concentrated under reduced pressure to remove methanol, and then added with water and ethyl acetate for extraction. An organic phase was separated, washed with a aqueous copper acetate solution, washed with saline, dried, concentrated and purified by column chromatography to afford product F902 (3 g).

### 3.3 Preparation of F903

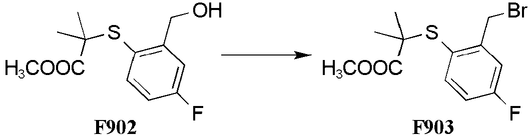

F902 (3 g, prepared according to the method in 3.2 as described above) and dichloromethane (20 mL) were added to a reaction flask, and then phosphorus tribromide (3.14 g) was added at -10°C for reaction for 0.5 h. Then the reaction was continued at room temperature until the starting materials was completely consumed. The reaction was quenched with water. The resulting reaction product was extracted with ethyl acetate. An organic phase was separated, washed, dried, concentrated and purified by column chromatography to afford product F903.

### 3.4 Preparation of F904

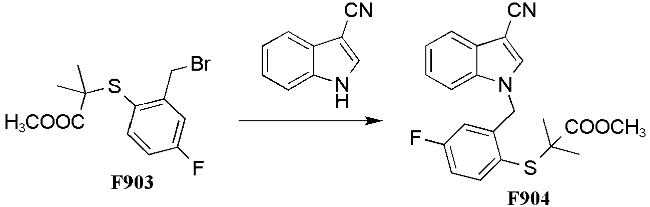

3-cyanoindole (1.4 g), sodium hydroxide (0.7 g) and dimethylformamide (25 mL) were added to a reaction flask and allowed to reaction for 1 h at room temperature. F903 prepared in the above step 3.3 was then added, and the reaction was continued at room temperature until the starting materials was completely consumed. Water and ethyl acetate were added for extraction. An organic phase was separated, washed, dried and concentrated and purified by column chromatography to afford product F904 (2 g).

### 3.5 Preparation of F-09

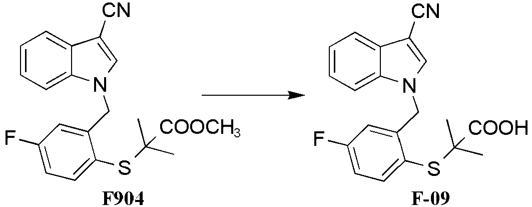

F904 (2 g, prepared according to the method in 3.4 as described above) and tetrahydrofuran (20 mL) were added to a reaction flask and dissolved, and then methanol (10 mL), water (10 mL) and lithium hydroxide (0.42 g) were added to allow reaction at 60°C for 1 h. Then dilute hydrochloric acid was added to adjust the pH to 4. The resulting reaction product was concentrated under reduced pressure, extracted with ethyl acetate, concentrated and purified by column chromatography to afford product F-09 (1.53 g). Mass spectrum: [M+H]⁺: 369.11; nuclear magnetism: ¹H NMR (400 MHz, DMSO-*d*₆) δ 12.86 (s, 1H), 8.34 (s, 1H), 7.70 (dd, *J* = 6.8, 1.7 Hz, 1H), 7.64 (dd, *J* = 8.6, 5.9 Hz, 1H), 7.54 (d, *J* = 7.3 Hz, 1H), 7.36-7.29 (m, 2H), 7.22 (td, *J* = 8.5, 2.9 Hz, 1H), 6.59 (dd, *J* = 9.8, 2.9 Hz, 1H), 5.70 (s, 2H), 1.46 (s, 6H).

### Example 4: Preparation of Compound F-12

### 4.1 Preparation of F1201

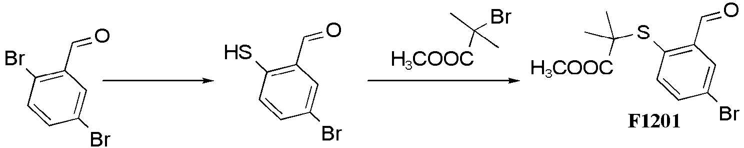

Sodium hydrosulfide hydrate (8.3 g), dimethylformamide (200 mL) and potassium carbonate (19.6 g) were added to a reaction flask and stirred, and 2,5-dibromobenzaldehyde (25 g) was then added for reaction at 70°C until thin-layer chromatography (TLC) indicated that the starting materials were completely consumed. Then, the reaction mixture was cooled. Methyl 2-bromoisobutyrate (18.8 g) was then added for reaction for 20 min at room temperature. Water and ethyl acetate were added for extraction. An organic phase was separated, washed with an aqueous copper acetate solution, washed with saline, dried, concentrated and purified by column chromatography to afford product F1201 (22.5 g).

### 4.2 Preparation of F1202

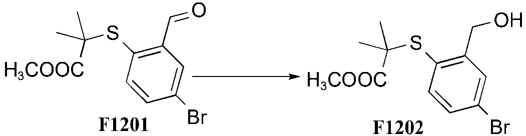

F1201 (22.5 g, prepared according to the method in 4.1 as described above) and methanol (50 mL) were added to a reaction flask, and then sodium borohydride (1.9 g) was added at -10°C. After the starting materials were completely consumed, the reaction was quenched with water. The resulting reaction product was concentrated under reduced pressure to remove methanol, added with water and ethyl acetate for extraction. An organic phase was separated, washed with an aqueous copper acetate solution, washed with saline, dried, concentrated and purified by column chromatography to afford product F1202 (15.9 g).

### 4.3 Preparation of F1203

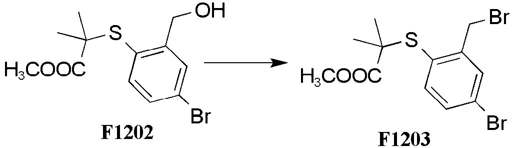

F1202 (15.9 g, prepared according to the method in 4.2 as described above) and dichloromethane (100 mL) were added to a reaction flask, and then phosphorus tribromide (13.5 g) was added at -10°C for reaction for 0.5 h. Then the reaction was continued at room temperature until the starting materials were completely consumed. The reaction was quenched with water, and the resulting reaction product was extracted with ethyl acetate. An organic phase was separated, washed, dried, concentrated and purified by column chromatography to afford product F1203.

### 4.4 Preparation of F1204

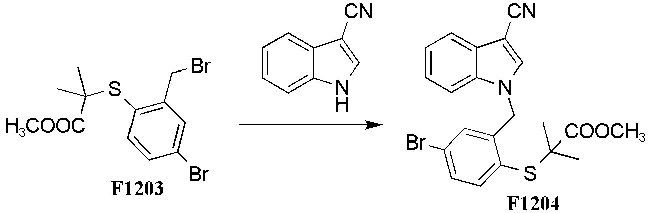

3-cyanoindole (6 g), sodium hydroxide (3 g) and dimethylformamide (100 mL) were added to a reaction flask and allowed to reaction for 1 h at room temperature. Then, F1203 obtained in the above step 4.3 was added for reaction for 0.5 h at room temperature. Water and ethyl acetate were added for extraction. An organic phase was separated, washed, dried, concentrated and purified by column chromatography to afford product F1204 (12.1 g).

### 4.5 Preparation of F1205

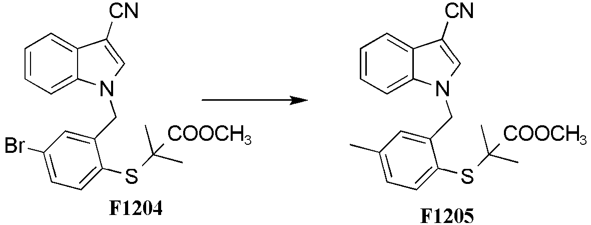

F1204 (3 g, prepared according to the method in 4.4 as described above), methylboronic acid (0.49 g), potassium carbonate (1.4 g), dioxane (30 mL) and water (6 mL) were added to a reaction flask, and then [1,1'-Bis(diphenylphosphino)ferrocene]palladium(II)dichloride (Pd(dppf)Cl₂, 0.29 g) was added under a nitrogen atmosphere. The resulting mixture was reacted at 100°C overnight under nitrogen. Then, the resulting reaction product was filtered and extracted with ethyl acetate. An organic phase was separated, washed, dried, concentrated and purified by column chromatography to afford product F1205 (1.8 g).

### 4.6 Preparation of F-12

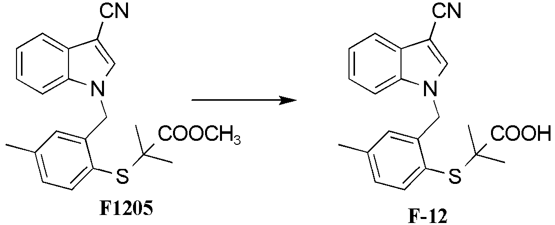

F1205 (1.8 g, prepared according to the method in 4.5 as described above) and tetrahydrofuran (20 mL) were added to a reaction flask and dissolved, and then methanol (10 mL), water (10 mL) and lithium hydroxide (0.4 g) were added. The resulting mixture was reacted at 60°C until the starting materials were completely consumed. Dilute hydrochloric acid was added to adjust the pH to 4. The resulting reaction product was concentrated, extracted with ethyl acetate, concentrated and purified by column chromatography to afford product F-12 (1.55 g). Mass spectrum: [M+H]⁺: 365.14; nuclear magnetism: ¹H NMR (400 MHz, DMSO-*d*₆) δ 12.64 (s, 1H), 8.31 (s, 1H), 7.69-7.66 (m, 1H), 7.53 (dd, *J* = 7.1, 1.3 Hz, 1H), 7.46 (d, *J* = 7.9 Hz, 1H), 7.34-7.26 (m, 2H), 7.14 (dd, *J* = 7.9, 1.4 Hz, 1H), 6.72 (d, *J* = 1.5 Hz, 1H), 5.66 (s, 2H), 2.17 (s, 3H), 1.42 (s, 6H).

### Example 5: Preparation of Compound F-14

### 5.1 Preparation of F1401

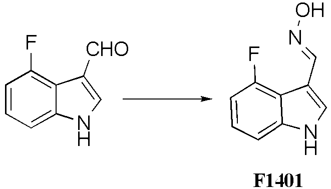

4-fluoro-1H-indole-3-carbaldehyde (0.815 g), hydroxylamine hydrochloride (0.416 g) and methanol (15 mL) were added to a reaction flask and allowed to reaction at 80°C until the starting materials were completely consumed. The resulting reaction product was concentrated under reduced pressure, and then added with water and ethyl acetate for extraction. An organic phase was separated, dried and concentrated to afford product F1401 (0.854 g).

### 5.2 Preparation of F1402

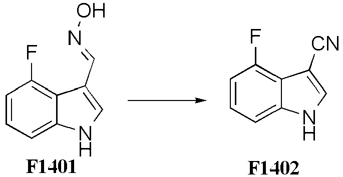

F1401 (0.845 g, prepared according to the method in 5.1 as described above) and thionyl chloride (5 mL) were added to a reaction flask and allowed to reaction for 30 min at 80°C. The resulting reaction product was cooled, concentrated, added with water and ethyl acetate for extraction. An organic phase was separated, washed and concentrated to afford product F1402 (0.660 g).

### 5.3 Preparation of F1403

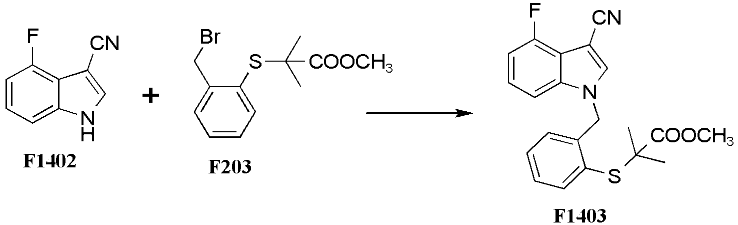

F1402 (0.320 g, prepared according to the method in 5.2 as described above), sodium hydroxide (80 mg) and dimethylformamide (5 mL) were added to a reaction flask and allowed to reaction for 10 min at 50°C. F203 (0.606 g, prepared according to the method in Example 1) was then added. After the reaction was continued for 30 min, the reaction was quenched with water. The resulting reaction product was extracted with ethyl acetate. An organic phase was dried and concentrated to afford product F1403 (0.911 g).

### 5.4 Preparation of F-14

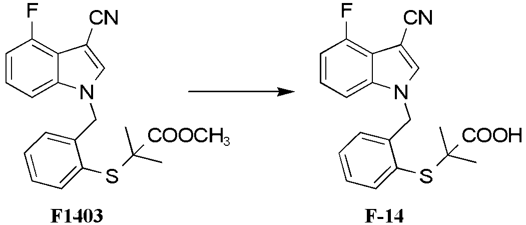

F1403 (0.911 g, prepared according to the method in 5.3 as described above), tetrahydrofuran (4 mL), methanol (2 mL), water (2 mL) and lithium hydroxide (0.168 g) were added to a reaction flask and allowed to reaction for 40 min at 50°C. Then, the reaction was quenched with water. Citric acid was added to adjust the pH to be acidic. The resulting reaction product was extracted with ethyl acetate. An organic phase was separated, dried, concentrated and purified by column chromatography to afford product F-14 (0.649 g). Mass spectrum: [M+H]⁺: 369.13; nuclear magnetism: ¹H NMR (400 MHz, DMSO-d6) δ 12.83 (br, 1H), δ 8.41 (s, 1H), 7.59-7.56 (m, 1H), 7.38-7.29 (m, 4H), 7.11-7.06 (m, 1H), 6.80-6.77 (m, 1H), 5.72 (s, 2H), 1.45 (s, 6H).

### Example 6: Preparation of Compound F-16

### 6.1 Preparation of F1601

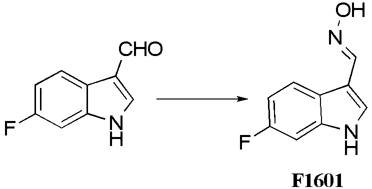

6-fluoro-1H-indole-3-carbaldehyde (1.63 g), hydroxylamine hydrochloride (0.83 g) and methanol (30 mL) were added to a reaction flask and allowed to reaction for 3 h at 80°C. The resulting reaction product was concentrated under reduced pressure, and then added with water and ethyl acetate for extraction. An organic phase was separated, dried and concentrated to afford product F1601 (1.684 g).

### 6.2 Preparation of F1602

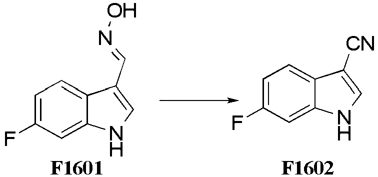

F1601 (1.684 g, prepared according to the method in 6.1 as described above) and thionyl chloride (5 mL) were added to a reaction flask and allowed to reaction for 30 min at 80°C. The resulting reaction product was concentrated, and then added with water and ethyl acetate for extraction. An organic phase was separated, washed, concentrated and purified by column chromatography to afford product F1602 (1.054 g).

### 6.3 Preparation of F1603

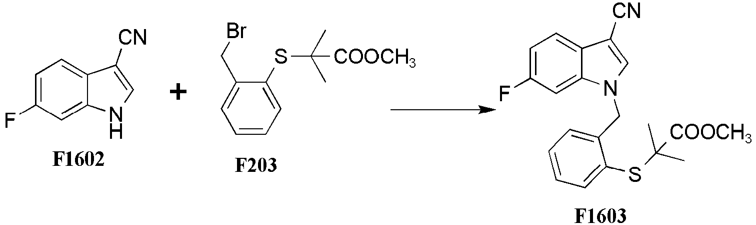

F1602 (0.320 g, prepared according to the method in 6.2 as described above), sodium hydroxide (80 mg) and dimethylformamide (5 mL) were added to a reaction flask and allowed to reaction for 10 min at 50°C. F203 (0.606 g, prepared according to the method in Example 1 as described above) was then added. After the reaction was continued for 30 min, the reaction was quenched with water. The resulting reaction product was extracted with ethyl acetate. An organic phase was dried and concentrated to afford crude product F1603 (0.900 g).

### 6.4 Preparation of F-16

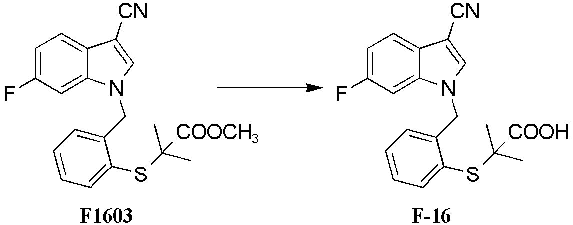

F1603 (0.900 g, prepared according to the method in 6.3 as described above), tetrahydrofuran (4 mL), methanol (2 mL), water (2 mL) and lithium hydroxide (0.168 g) were added to a reaction flask and allowed to reaction for 40 min at 50°C. The reaction was quenched with water. Citric acid was added to adjust the pH to be acidic. Then, the resulting reaction product was extracted with ethyl acetate. An organic phase was separated, dried, concentrated and purified by column chromatography to afford product F-16 (0.820 g). Mass spectrum: [M+H]⁺: 369.13; nuclear magnetism: ¹H NMR (400 MHz, DMSO-d6) δ12.85 (br, 1H), 8.31 (s, 1H), 7.74-7.70 (m, 1H), 7.60-7.57 (m, 1H), 7.46 (dd, J=10Hz, 2.4 Hz, 1H), 7.37-7.34 (m, 2H), 7.18 (td, J=10Hz, 2.4Hz, 1H), 6.80-6.77 (m, 1H), 5.67 (s, 2H), 1.46 (s,6H).

### Example 7: Preparation of Compound F-17

### 7.1 Preparation of F1701

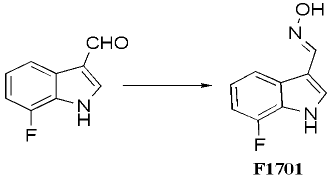

7-fluoro-1H-indole-3-carbaldehyde (0.490 g), hydroxylamine hydrochloride (0.333 g) and methanol (10 mL) were added to a reaction flask and allowed to reaction at 80°C until the starting materials were completely consumed. The resulting reaction product was concentrated under reduced pressure, and then added with water and ethyl acetate for extraction. An organic phase was separated, dried and concentrated to afford product F1701 (0.600 g).

### 7.2 Preparation of F1702

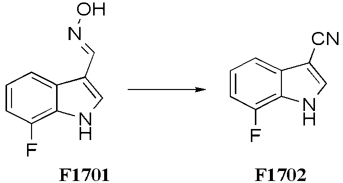

F1701 (1.085 g, prepared according to the method in 7.1 as described above) and thionyl chloride (5 mL) were added to a reaction flask and allowed to reaction for 30 min at 80°C. The resulting reaction product was concentrated, added with water and ethyl acetate for extraction. An organic phase was separated, washed and concentrated to afford product F1702 (1.01 g).

### 7.3 Preparation of F1703

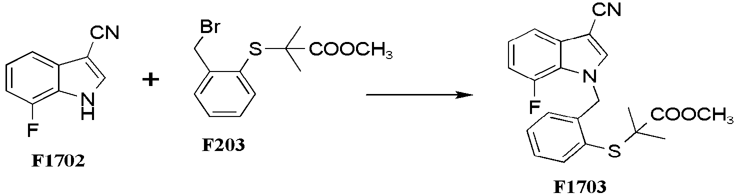

F1702 (0.82 g, prepared according to the method in 7.2 as described above), sodium hydroxide (0.24 g) and dimethylformamide (8 mL) were added to a reaction flask and allowed to reaction for 10 min at 50°C. Then, F203 (1.56 g, prepared according to the method in Example 1 as described above) was added. After the reaction was continued under stirring for 1 h, the reaction was quenched with water. The resulting reaction product was extracted with ethyl acetate. An organic phase was washed, dried and concentrated to afford crude product F1703 (2 g).

### 7.4 Preparation of F-17

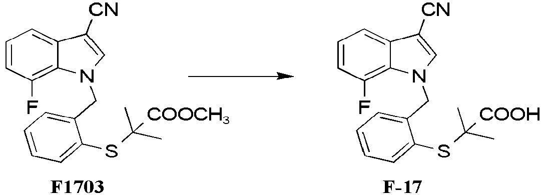

F1703 (2 g, prepared according to the method in 7.3 as described above), tetrahydrofuran (4 mL), methanol (2 mL) and water (2 mL) were added to a reaction flask. Lithium hydroxide was added to adjust the pH to 14. After the reaction was conducted for 40 min at 50°C, the reaction was quenched with water. Citric acid was added to adjust the pH to be acidic. Then, the resulting reaction product was extracted with ethyl acetate. An organic phase was separated, dried, concentrated and purified by column chromatography to afford product F-17 (0.449 g). Mass spectrum: [M+H]⁺: 369.20; nuclear magnetism: ¹H NMR (400 MHz, DMSO-d6) δ12.85 (br, 1H), 8.35 (s, 1H), 7.57-7.51 (m, 2H), 7.34-7.24 (m, 3H), 7.15-7.10 (m, 1H), 6.57-6.59 (m,1H), 5.82 (s,2H), 1.44 (s, 6H).

### Example 8: Preparation of Compound F-20

### 8.1 Preparation of F2001

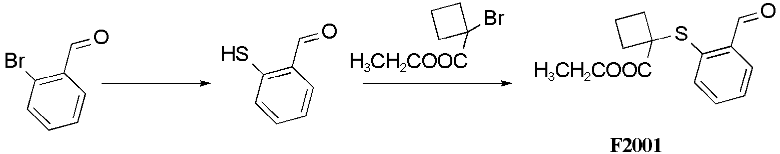

2-bromobenzaldehyde (15.00 g), sodium hydrosulfide (6.49 g), potassium carbonate (16.79 g) and dimethylformamide (80 mL) were added to a reaction flask and allowed to reaction at 70°C, until the starting materials were almost completely consumed. The reaction mixture was cooled to room temperature.

Ethyl 1-bromocyclobutanecarboxylate (16.77 g) was added for reaction at room temperature, until the starting materials were almost completely consumed. Then, the reaction was quenched with an aqueous copper acetate solution. The resulting reaction product was extracted with ethyl acetate, washed with saturated saline, and dried over anhydrous sodium sulfate, and the organic phase was collected, concentrated and purified by column chromatography to afford product F2001 (12.41 g).

### 8.2 Preparation of F2002

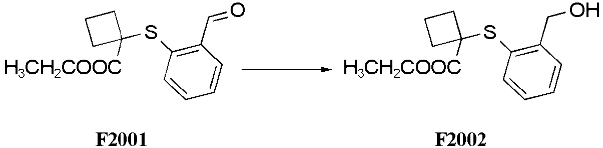

F2001 (12.41 g, prepared according to the method in 8.1 as described above) and methanol (60 mL) were added to a reaction flask and stirred below 0°C, and then sodium borohydride (0.888 g) was added. After the starting materials were completely consumed, the reaction was quenched with water. Methanol was removed by concentration under reduced pressure. Citric acid was added to adjust the pH to 6. Then, the resulting reaction product was added with an aqueous copper acetate solution and ethyl acetate for extraction, washed with saturated saline, and dried over anhydrous sodium sulfate, and organic phase was collected, concentrated and purified by column chromatography to afford product F2002 (10.51 g).

### 8.3 Preparation of F2003

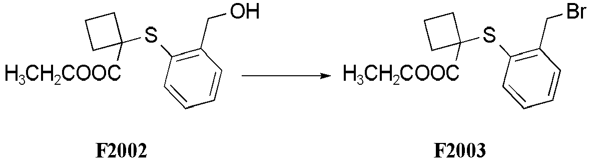

F2002 (2.66 g, prepared according to the method in 8.2 as described above) and dichloromethane (18 mL) were added to a reaction flask, and then phosphorus tribromide (2.70 g) was added at -15°C. The resulting mixture was stirred for reaction, until the starting materials were completely consumed. Then, the reaction was quenched with water. The aqueous phase and organic phase were separated, and the aqueous phase was extracted with dichloromethane. The organic phases were combined, washed with semi-saturated saline, dried over anhydrous sodium sulfate, concentrated, and purified by column chromatography to afford product F2003 (1.56 g).

### 8.4 Preparation of F2004

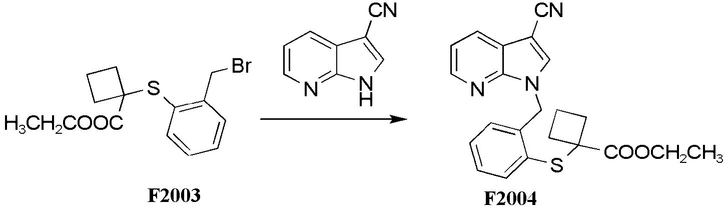

3-cyano-7-azaindole (0.678 g), F2003 (1.56 g, prepared according to the method in 8.3 as described above) and dimethylformamide (6 mL) were added to a reaction flask. The sodium hydroxide (0.227 g) was then added at a controlled temperature of about 50°C for reaction for 1 h under stirring. After that, the reaction was quenched with water. Citric acid was added to adjust the pH to 4. The resulting reaction product was extracted with ethyl acetate, and dried over anhydrous sodium sulfate, and the organic phase was collected, and concentrated to afford crude product F2004 (2.20 g).

### 8.5 Preparation of F-20

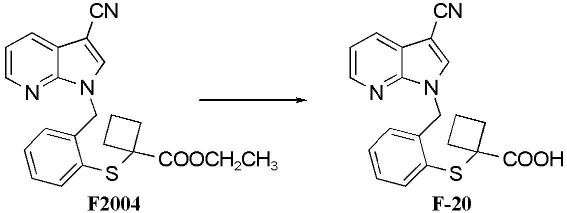

F2004 (2.20 g, prepared according to the method in 8.4 as described above), methanol (2 mL), tetrahydrofuran (4 mL) and water (2 mL) were added to a reaction flask. Lithium hydroxide was added to adjust the pH to approximately 14. The resulting mixture was allowed to reaction for 1 h at 50°C under stirring. The reaction solution was concentrated to dryness, added with water and ethyl acetate for extraction. An aqueous phase was separated, acidified with dilute hydrochloric acid to pH 5-6, and then extracted with ethyl acetate. An organic phase was separated, concentrated and purified by column chromatography to afford product F-20 (0.854 g). Mass spectrum: [M+H]⁺: 364.14; nuclear magnetism: ¹H NMR (400MHz, DMSO-d6) δ12.90 (br, 1H), 8.48 (s, 1H), 8.45 (dd, J=4.8, 1.6Hz, 1H), 8.21 (dd, J=8.0Hz, 1.6Hz, 1H), 7.38-7.36 (m, 2H), 7.28 (td, J=7.6Hz, 1.6Hz, 1H), 7.20 (td, J=7.6Hz, 1.6Hz, 1H), 6.77 (d, J=8.0Hz, 1H), 5.68 (s, 2H), 2.69-2.61 (m, 2H), 2.20-2.13 (m, 3H), 1.92-1.82 (m, 1H).

### Example 9: Preparation of Compound F-26

### 9.1 Preparation of F2601

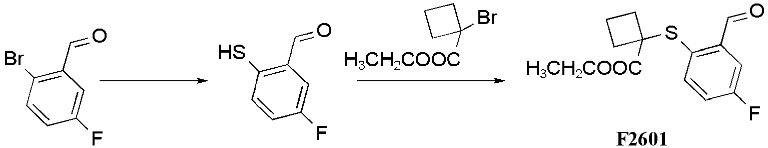

2-bromo-5-fluorobenzaldehyde (24.7 g), sodium hydrosulfide (10.3 g), potassium carbonate (25 g) and dimethylformamide (250 mL) were added to a reaction flask and allowed to reaction for 30 min at 70°C. The resulting reaction mixture was cooled to room temperature. Ethyl 1-bromocyclobutanecarboxylate (27.56 g) was added, and the reaction was continued for 30 min, during which the reaction system changed from brown-black to red. After the starting materials were almost completely consumed, the reaction was quenched with an aqueous copper acetate solution. The resulting reaction product was extracted with ethyl acetate, washed with a semi-saturated saline, and dried over anhydrous sodium sulfate, and the organic phase was collected, concentrated and purified by column chromatography to afford product F2601 (18 g).

### 9.2 Preparation of F2602

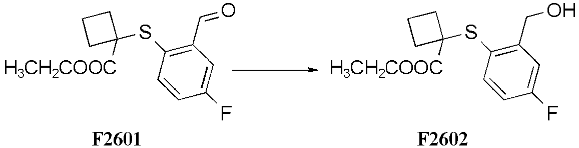

F2601 (18 g, prepared according to the method in 9.1 as described above) and methanol (40 mL) were added to a reaction flask and stirred below 0°C. Sodium borohydride (1.69g) was then added for reaction for 2 h. Then, the reaction was quenched with water. Methanol was removed by concentration under reduced pressure. Citric acid was added to the residue to adjust the pH to approximately 6. Then, the resulting reaction product was added with an aqueous copper acetate solution and ethyl acetate for extraction, washed with semi-saturated saline, and dried over anhydrous sodium sulfate, and the organic phase was collected, and concentrated to afford product F2602 (13.21 g).

### 9.3 Preparation of F2603

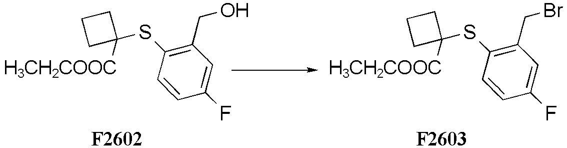

F2602 (10.80 g, prepared according to the method in 9.2 as described above) and dichloromethane (55 mL) were added to a reaction flask, and then phosphorus tribromide (10.28 g) was added at -15°C for reaction under stirring, until the starting materials were completely consumed. Then, the reaction was quenched with water. Aqueous phase and organic phase were separated, and the aqueous phase was extracted with dichloromethane. The organic phases were combined, washed with semi-saturated saline, dried over anhydrous sodium sulfate, concentrated, and purified by column chromatography to afford product F2603 (6.15 g).

### 9.4 Preparation of F2604

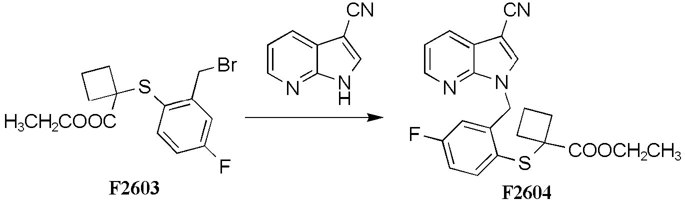

3-cyano-7-azaindole (0.716 g), F2603 (1.606 g, prepared according to the method in 9.3 as described above) and dimethylformamide (5 mL) were added to a reaction flask. The sodium hydride (0.300 g) was then added at a controlled temperature of about 50°C for reaction for 1 h under stirring. After that, the reaction was quenched with water. Citric acid was added to adjust the pH to 4. The resulting reaction product was extracted with ethyl acetate, and dried over anhydrous sodium sulfate, and the organic phase was collected, and concentrated to afford crude product F2604 (2.87 g).

### 9.5 Preparation of F-26

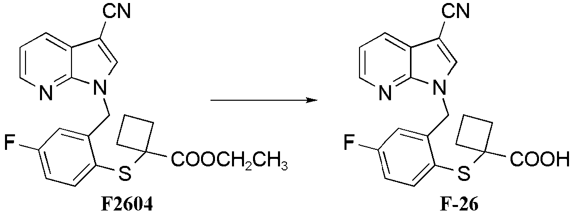

F2604 (2.87 g, prepared according to the method in 9.4 as described above), ethanol (3 mL), tetrahydrofuran (6 mL) and water (3 mL) were added to a reaction flask. Lithium hydroxide was added to adjust the pH to 14. The resulting mixture was allowed to reaction for 1 h at 50°C under stirring. The reaction solution was concentrated to dryness, added with water and ethyl acetate for extraction. An aqueous phase was separated, acidified with dilute hydrochloric acid to pH 5-6, and then extracted with ethyl acetate. An organic phase was separated, concentrated and purified by column chromatography to afford product F-26 (0.497 g). Mass spectrum: [M+H]⁺: 382.15; nuclear magnetism: ¹H NMR (400MHz, DMSO-d6) δ12.91 (br, 1H), 8.49 (s, 1H), 8.46 (dd, J=4.4Hz, 1.2Hz, 1H), 8.21 (dd, J=8.0Hz, 1.2Hz, 1H), 7.52-7.48 (m, 1H), 7.41-7.36 (m, 1H), 7.21 (td, J=8.4Hz, 2.8Hz, 1H), 6.68 (dd, J=10.0Hz, 2.4Hz, 1H), 5.71 (s, 2H), 2.65-2.58 (m, 2H), 2.21-2.09 (m, 3H), 1.89-1.84 (m, 1H).

### Example 10: Preparation of other compounds

Based on the methods described in Examples 1-9, some compounds in the present disclosure were obtained, see in Table 1.

**Table 1 Examples of some compounds of the present disclosure**

| Compound number | Structural formula | Mass spectrum | Nuclear magnetism |
|---|---|---|---|
| F-05 | 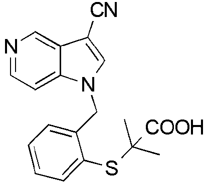 | [M+H]⁺ 352.12 | ¹H NMR (400 MHz, DMSO-*d*₆) δ12.83 (br, 1H), δ8.99 (s, 1H), δ8.43 (s, 1H), 8.41-8.40 (m,1H), 7.60-7.57 (m, 2H), 7.37-7.33 (m, 2H), 6.90-6.78 (m, 1H), 5.72 (s, 2H), 1.44 (s, 6H). |
| F-08 | 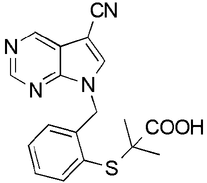 | [M+H]⁺ 353.08 | ¹H NMR (400 MHz, DMSO-*d*₆) δ 12.85 (s, 1H), 9.28 (s, 1H), 9.01 (s, 1H), 8.58 (s, 1H), 7.59-7.53 (m, 1H), 7.37-7.31 (m, 2H), 6.94-6.88 (m, 1H), 5.77 (s, 2H), 1.44 (s, 6H). |
| F-10 | 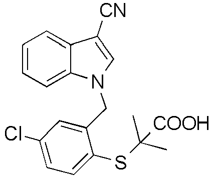 | [M+H]⁺ 385.04 | ¹H NMR (400 MHz, DMSO-*d*₆) δ 12.91 (s, 1H), 8.34 (s, 1H), 7.71 (dd, *J* = 6.9, 1.5 Hz, 1H), 7.60 (d, *J* = 8.3 Hz, 1H), 7.55 (d, *J* = 7.4 Hz, 1H), 7.44 (dd, *J* = 8.3, 2.4 Hz, 1H), 7.37-7.29 (m, 2H), 6.83 (d, *J* = 2.4 Hz, 1H), 5.69 (s, 2H), 1.47 (s, 6H). |
| F-11 | 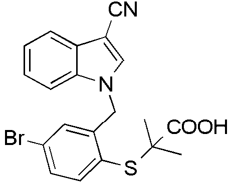 | [M-H] 427.01, 429.03 | ¹H NMR (400 MHz, DMSO-*d*₆) δ 12.93 (s, 1H), 8.33 (s, 1H), 7.70 (dd, *J* = 6.9, 1.3 Hz, 1H), 7.59-7.53 (m, 2H), 7.51 (d, *J* = 8.3 Hz, 1H), 7.39-7.27 (m, 2H), 6.99 (d, *J* = 2.2 Hz, 1H), 5.68 (s, 2H), 1.46 (s, 6H). |
| F-13 | 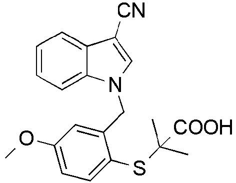 | [M+H]⁺ 381.10 | ¹H NMR (400 MHz, DMSO-*d*₆) δ 12.74 (br, 1H), δ 8.33 (s, 1H), 7.70-7.67 (d, J=7.2Hz, 1H), 7.56-7.50 (m, 2H), 7.35-7.27 (m,2H), 6.93 (dd,J=8.4Hz, 3.2Hz, 1H), 6.32 (d, *J* = 2.8Hz,1H), 5.64 (s, 2H), 3.65 (s, 3H), 1.42 (s,6H). |
| F-27 | 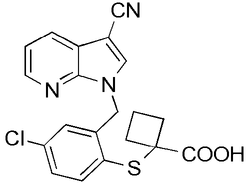 | [M+H]⁺ 398.11 | ¹H NMR (400MHz,DMSO-*d*₆) δ12.97 (br,1H), 8.49 (s,1H), 8.46 (dd, J=5.2Hz, 2.0Hz, 1H), 8.21 (dd, J=8.0Hz, 1.2Hz, 1H), 7.43-7.37 (m, 3H), 6.91 (d,J=2.0Hz,1H), 5.67 (s,2H), 2.70-2.63 (m,2H), 2.22-2.09 (m,3H), 1.91-1.87 (m,1H). |
| F-28 | 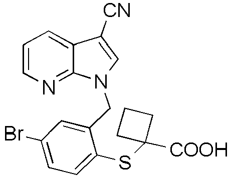 | [M+H]⁺ 442.10, 444.08 | ¹H NMR (400MHz,DMSO-*d*₆) δ12.98 (br,1H), 8.49 (s,1H), 8.47 (dd, J=4.4Hz, 1.2Hz, 1H), 8.21 (dd, J=8.0Hz, 1.2Hz, 1H), 7.53 (dd, J=8.4Hz, 2.4Hz, 1H), 7.39 (dd, J=8.0Hz, 5.2Hz, 1H), 7.31 (d, J=8.8Hz, 1H), 7.05 (d, J=2.0Hz, 1H), 5.66 (s, 2H), 2.70-2.63 (m, 2H), 2.21-2.09 (m, 3H), 1.92-1.86 (m, 1H). |
| F-30 | 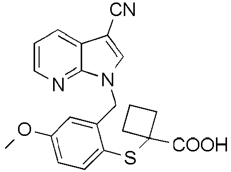 | [M+H]⁺ 394.13 | ¹H NMR (400MHz,DMSO-*d*₆) δ 12.96 (br, 1H), 8.46 (dd, J=5.2Hz, 2.0Hz, 1H), 8.41 (s, 1H), 8.18 (dd, J=8.0Hz, 2.0Hz, 1H), 7.37 (m, 1H), 6.93-6.89 (m, 2H), 6.82-6.78 (m, 1H), 5.58 (s, 2H), 3.71 (s, 3H), 2.71-2.65 (m, 2H), 2.20-2.06 (m, 3H), 1.92-1.84 (m, 1H). |

### Example 11: Drug efficacy test

Inhibitory activity of each compound against urate transporter URAT1 was tested.

Compounds: the compounds of the present disclosure were prepared according to the methods in examples; Verinurad was purchased commercially; and CDER167 was prepared according to the method described in the literature (doi: 10.1038/s41401-021-00640-5).

Cells: Flp-In-T-REx-293-hURAT1 cells capable of expressing human urate transporter 1 (hURAT1), were purchased from Pharmaron Beijing Co., Ltd., China or can be generated (e.g., by transfecting hURAT1 genes into commercially available Flp-In-T-REx-293 cells according to the method described in the literature doi: 10.1124/dmd.106.012187, and the expression of the hURAT1 genes can be verified by methods such as Western blotting).

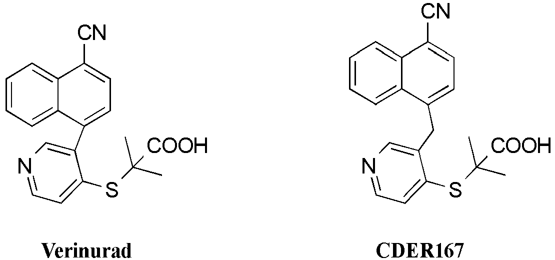

The Flp-In-T-REx-293-hURAT1 cells were cultured in a Dulbecco's Modified Eagle Medium (DMEM) in 75 cm² flasks under 5% CO₂ and 37°C, and passaged every two days. When reaching a confluency of 80%-90% during routine culture, the cells were digested and then centrifuged at 200 × g for 5 min to collect the cells. The cells were resuspended in the DMEM and then counted, and subsequently prepared into a cell suspension with a proper density. The cells were seeded into a 96-well plate and cultured in a 37°C and 5% CO₂ incubator for 24 h. The culture medium was removed, and DMEM containing 200 ng/mL doxycycline was added. The cells were then placed back into the 37°C and 5% CO₂ incubator and cultured for 18 h. The test compounds were serially diluted 3-fold in DMSO starting from 10 mmol/L to prepare 10 stock solutions at varying concentrations. A buffer solution was prepared, which contained 125 mmol/L sodium gluconate, 4.8 mmol/L potassium gluconate, 5.6 mmol/L glucose, 1.2 mmol/L potassium dihydrogen phosphate, 1.2 mmol/L magnesium sulfate, 1.3 mmol/L calcium gluconate and 25 mmol/L HEPES (pH = 7.4). 1.2 µL of each test compound stock solution was mixed with 119 µL of buffer to form an assay buffer. After induction for 16 h, the culture medium was removed, and the cells were washed with a prewarmed buffer and incubated for 10 min at 37°C. The buffer was then removed, and an assay buffer containing 50 µmol/L ¹⁴C-uric acid and the test compound was added to the cells, and then the cells were incubated at 37°C for 5 min. The buffer was removed, the cells were washed three times with a precool buffer. Subsequently, 100 mmol/L NaOH was added to lyse the cells for 20 min. The lysate was transferred to a 96-well plate, and then 200 µL of UltimaGold XR scintillation cocktail was added, and radioactivity was measured using a MicroBeta² (PerkinElmer). The half-maximal inhibitory concentration (IC₅₀) of each test compound was calculated via GraphPad Prism 8 software using a nonlinear regression fitting equation. The IC₅₀ values were categorized by the following grades: Grade A: < 100 nmol/L; Grade B: 100-500 nmol/L; Grade C: 500-1000 nmol/L; Grade D: > 1000 nmol/L. The results are shown in Table 2.

**Table 2 Inhibitory activity (IC₅₀) of some compounds in the present disclosure against urate transporter**

| Compound number | IC₅₀ (nmol/ L) | Compound number | IC₅₀ (nmol/L) |
|---|---|---|---|
| F-02 | B | F-14 | B |
| F-05 | A | F-16 | B |
| F-07 | A | F-17 | B |
| F-08 | B | F-20 | B |
| F-09 | B | F-26 | B |
| F-10 | B | F-27 | B |
| F-11 | B | F-28 | B |
| F-12 | B | F-30 | B |
| F-13 | B | CDER167 | D |
| Verinurad | C | | |

This experiment shows that compared to similar compounds, the compounds of the present disclosure demonstrate stronger inhibitory activity against URAT1.

### Example 12: Toxicity test

The toxicity of each compound was evaluated following 28-day repeated administration in mice.

Method: ICR mice that passed quarantine were selected, with equal numbers of males and females. The mice were randomly grouped based on body weight and sex, with 8 mice per group. The test compounds included the compounds of the present disclosure and a positive drug Verinurad. The test compounds were administered to the animals via intragastric gavage at a dosage of 200 mg/kg, once daily for 28 consecutive days. Detailed clinical observations were performed after each administration, with at least 2 h observation following the first administration or upon detection of abnormalities. In addition, cage-side observations were conducted once daily in the afternoon. Observations included clinical signs (animal appearance, behavior, diet, response to stimuli, secretions and excretions) and mortality (time of death or time of discovery of death, pre-mortem signs, etc.). In addition, the symptoms of animals, and the onset, severity, and duration of any observed clinical symptoms of animals were recorded. On Day 28 of administration, the mice were euthanized immediately after blood collection was completed and subjected to necropsy. Livers and left kidneys were collected for paraffin embedding, sectioning and hematoxylin-eosin (HE) staining, followed by histopathological examination to observe tissue lesions. Special attention was paid to the degeneration and inflammatory changes in glomeruli and renal tubules, as well as the presence of urea crystals. The animals euthanized due to found moribund or the animals dead during the experiment were subjected to necropsy promptly.

Results: following 28-day repeated administration, compounds F-02, F-05, F-07, F-08, F-09, F-10, F-11, F-12, F-13, F-14, F-16, F-17, F-20, F-26, F-27, F-28 and F-30 showed no hepatic or renal toxicity upon histopathological examination. In contrast, Verinurad induced renal toxicity in 3 out of 8 mice (37.5%) after 28-day repeated administration.

This experiment shows that compared to similar compounds, the compounds of the present disclosure exhibit an improved safety profile.

The present disclosure has been described in detail by way of general descriptions and specific embodiments. On this basis, those skilled in the art may make certain changes or improvements thereto. All such changes or improvements made without departing from the scope of protection of the present disclosure shall fall within the scope of the content to be protected by the present disclosure.

## Claims

1. A compound of Formula I, or a pharmaceutically acceptable salt thereof, 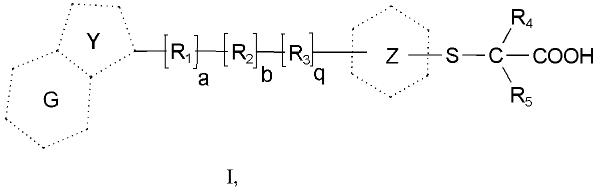 wherein ring G is a 6-membered ring, ring Y is a 5-membered ring, ring Z is a 6-membered ring, and the ring G and the ring Y are fused through two atoms; the ring G, the ring Y or the ring Z is optionally substituted; R₁, R₂ and R₃ are each independently -CH₂- or -S-; a, b and q are each independently 0, 1, 2 or 3, and a, b and q are not all 0; R₄ and R₅ are each independently alkyl or alkoxy, and R₄ and R₅ optionally form a ring together or not.

2. The compound according to claim 1, wherein the ring G, the ring Y or the ring Z is each independently a carbocyclic ring or a heterocyclic ring.

3. The compound according to claim 1, wherein at least one of the ring G and the ring Y is a heterocyclic ring.

4. The compound according to claim 1, wherein the ring G, the ring Y or the ring Z is each independently a saturated ring or an unsaturated ring.

5. The compound according to claim 1, wherein at least one of the ring G and the ring Y is an unsaturated ring.

6. The compound according to claim 1, wherein the compound of Formula I is represented by Formula I-I: 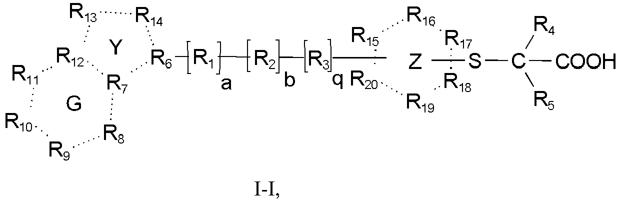 wherein ring G, ring Y, ring Z, R₁, R₂, R₃, R₄, R₅, a, b and q are as defined in claim 1; R₆, R₇ and R₁₂ are each independently -C- or -N-; R₈, R₉, R₁₀, R₁₁, R₁₃, R₁₄, R₁₅, R₁₆, R₁₇, R₁₈, R₁₉ and R₂₀ are each independently -C-, -N-, -S- or -O-.

7. The compound according to claim 6, wherein at least one of R₆, R₇, R₈, R₉, R₁₀, R₁₁, R₁₂, R₁₃ and R₁₄ is -N-.

8. The compound according to claim 6, wherein R₆ is -N-.

9. The compound according to claim 6, wherein the ring Y is an unsaturated ring.

10. The compound according to claim 1, wherein the compound of Formula I is represented by Formula I-II: 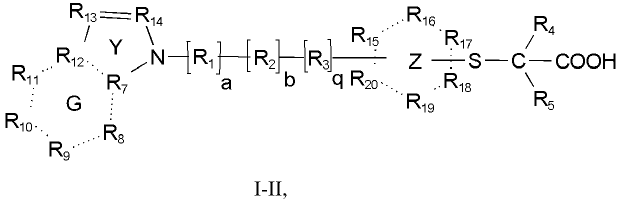 wherein ring G, ring Z, R₁, R₂, R₃, R₄, R₅, R₇, R₈, R₉, R₁₀, R₁₁, R₁₂, R₁₅, R₁₆, R₁₇, R₁₈, R₁₉, R₂₀, a, b and q are as defined in claim 6; the ring G, the ring Y or the ring Z is optionally substituted; and R₁₃ and R₁₄ are each independently -C- or -N-.

11. The compound according to claim 10, wherein 0 to 2 members selected from the group consisting of R₁₃, R₁₄, R₇ and R₁₂ are -N-.

12. The compound according to claim 10, wherein the ring Y is pyrrole, imidazole, pyrazole or triazole.

13. The compound according to claim 1, wherein the ring G is an unsaturated 6-membered ring.

14. The compound according to claim 1, wherein the compound of Formula I is represented by Formula I-III: 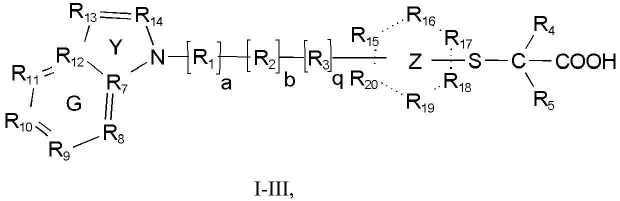 wherein ring Z, R₁, R₂, R₃, R₄, R₅, R₁₅, R₁₆, R₁₇, R₁₈, R₁₉, R₂₀, a, b and q are as defined in claim 10, ring G, ring Y or the ring Z is optionally substituted; and R₇, R₈, R₉, R₁₀, R₁₁, R₁₂, R₁₃ and R₁₄ are each independently -Cor -N-.

15. The compound according to claim 14, wherein 0 to 3 members selected from the group consisting of R₈, R₉, R₁₀ and R₁₁ are -N-.

16. The compound according to claim 14, the ring G is benzene, pyridine, pyridazine, pyrimidine, pyrazine or triazine.

17. The compound according to claim 1, wherein the ring Z is an unsaturated 6-membered ring.

18. The compound according claim 1, wherein the compound of Formula I is represented by Formula I-IV: 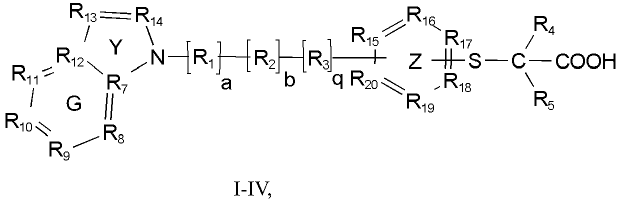 wherein R₁, R₂, R₃, R₄, R₅, R₇, R₈, R₉, R₁₀, R₁₁, R₁₂, R₁₃, R₁₄, a, b and q are as defined in claim 14, ring G, ring Y or ring Z is optionally substituted; and R₁₅, R₁₆, R₁₇, R₁₈, R₁₉ and R₂₀ are each independently -C- or -N-.

19. The compound according to claim 18, wherein 0 to 3 members selected from the group consisting of R₁₅, R₁₆, R₁₇, R₁₈, R₁₉ and R₂₀ are -N-.

20. The compound according to claim 18, wherein the ring Z is benzene, pyridine, pyridazine, pyrimidine, pyrazine or triazine.

21. The compound according to claim 1, wherein R₁ is -CH₂-, R₂ is -S-, R₃ is -CH₂-, a and q are independently 0, 1, 2 or 3, and b is 1.

22. The compound according to claim 1, wherein R₂ is -CH₂-, b is 1, 2 or 3, a is 0, and q is 0.

23. The compound according to claim 1, wherein the compound of Formula I is represented by Formula I-V: 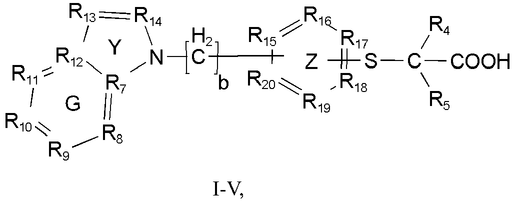 wherein R₄, R₅, R₇, R₈, R₉, R₁₀, R₁₁, R₁₂, R₁₃, R₁₄, R₁₅, R₁₆, R₁₇, R₁₈, R₁₉ and R₂₀ are as defined in claim 18, and ring G, ring Y or ring Z is optionally substituted; and b is 1, 2 or 3.

24. The compound according to claim 1, wherein the ring G, the ring Y or the ring Z is optionally substituted with one or more of alkyl, alkoxy, halogen, cyano, hydroxyl or carboxyl.

25. The compound according to claim 1, wherein the ring Y is substituted.

26. The compound according to claim 1, wherein the compound of Formula I is represented by Formula I-VI: 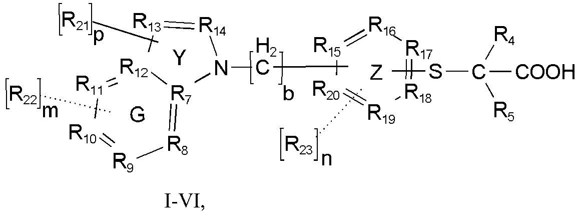 wherein R₄, R₅, R₇, R₈, R₉, R₁₀, R₁₁, R₁₂, R₁₃, R₁₄, R₁₅, R₁₆, R₁₇, R₁₈, R₁₉, R₂₀ and b are as defined in claim 23; ring Y is substituted with R₂₁, ring G is optionally substituted with R₂₂, and ring Z is optionally substituted with R₂₃; R₂₁ is alkyl, alkoxy, halogen or cyano; R₂₂ and R₂₃ are each independently alkyl, alkoxy or halogen; and p is 1 or 2, and m and n are each independently 0, 1, 2, 3 or 4.

27. The compound according to claim 1, wherein R₄ and R₅ do not form a ring together, and R₄ and R₅ are each independently C₁-C₅ alkyl.

28. The compound according to claim 1, wherein R₄ and R₅ form a ring together, and R₄ and R₅ form cyclopropyl, cyclobutyl, cyclopentyl or cyclohexyl together.

29. A compound of Formula II: 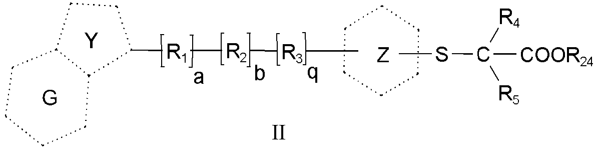 wherein ring Y, ring G, ring Z, R₁, R₂, R₃, R₄, R₅, a, b and q are as defined in claim 1; and R₂₄ is alkyl.

30. Use of the compound according to any one of claim 1-29 in the preparation of a drug for lowering a uric acid level in vivo or in the preparation of a drug for treating a disease associated with an abnormal uric acid level in vivo.

31. The use according to claim 30, wherein the disease is hyperuricemia, gout or renal failure accompanied by an abnormal uric acid level.
